# EUROPEAN PATENT APPLICATION

(11) **EP 1 588 722 A1**
(43) Date of publication of application: **26.10.2005**
(21) Application number: 04702784.2
(22) Date of filing: 16.01.2004
(51) Int. Cl.: A61L 15/00, A61F 13/00

(54) **HEMOSTATIC MATERIALS**

(30) Priority: 20.01.2003 JP 2003010494
(71) Applicant: Juridical Foundation The Chemo-Sero-Therapeutic Research Institute, Kumamoto-ken 860-8568 (JP)
(72) Inventor: UCHIDA, Takanori, Kumamoto-shi, Kumamoto-ken 860-8568 (JP); SHINYA, Noriko, Kikuchi-shi, Kumamoto-ken 869-1298 (JP); KAETSU, Hiroshi, Kumamoto-shi, Kumamoto-ken 860-8568 (JP)
(74) Representative: Vossius & Partner
(86) International application number: PCT/JP2004/000291
(87) International publication number: WO 2004/064878

(57) **Abstract**

A safe and effective hemostatic material is provided. A hemostatic material comprising as an effective ingredient thrombin and fibrinogen characterized in that a bioabsorbable synthetic nonwoven fabric is used as a supporting material. The use of the hemostatic material of the present invention allows for quick and thorough hemostasis for both projectile bleeding and exudative bleeding.

## Description

### TECHNICAL FIELD OF THE INVENTION

The present invention relates to a hemostatic material comprising as an effective ingredient thrombin and fibrinogen characterized in that a bioabsorbable synthetic nonwoven fabric is used as a supporting material.

### BACKGROUND OF THE INVENTION

Hemostatic management is very important on the clinical scene. In particular, in case of surgical operation, hemostatic manipulation is one of the most momentous tasks requiring much labor and time. As an exemplary, it is reported that a little longer two hours out of about five hours of operation may be used for hemostatic manipulation in a radical operation of tetralogy of Fallot. Thus, shortening of time for hemostatic manipulation will.much shorten time for operation.

Moreover, for hemostasis are important not only shortening of time for hemostasis but also suppression of a loss of blood to a minimum extent before hemostasis is achieved as well as thorough hemostasis. Bleeding, if in excess, will be lethal or cause severe complications subsequently. If hemostasis is not thoroughly attained, bleeding will repeatedly occur during or after operation. Recurrence of bleeding during operation even at a low degree may prevent a visual field for surgical operation to render easy manipulation more difficult. Recurrence of bleeding after operation may increase drainage from a drainage tube and liability to infection to thereby make postoperative management difficult. In worst cases, surgical operation will become necessary for hemostasis. In the field of cardiovascular surgery, there is a report that 3% of patients are forced to undergo surgical operation for hemostasis. Accordingly, shortening of time for operation and suppression of loss of blood as well as thorough hemostasis have been earnestly desired so that physicians' burden and stress of patients may be lowered.

A method for hemostasis commonly used includes
(1) a method for hemostasis using a mechanical means and
(2) a method for hemostasis by accelerating topical coagulation. Usually, a topical hemostatic or a filling agent is used for topical hemostasis in case that a method using a mechanical means such as ligature or suture may not effectively be applied. When a hemostatic is used, collagen fiber, dry thrombin powder, oxidized cellulose, gelatins, fibrin adhesives, etc. may be delivered or adhered to the bleeding site, which is then compressed for five to ten minutes. With the currently used method for hemostasis, there are problems as described above that substantial time for hemostasis must be taken from time for surgical operation, that ligature may be slipped off, that recurrence of bleeding may occur due to non-thorough hemostasis, and the like.

### DISCLOSURE OF THE INVENTION

### (Technical Problem to be Solved by the Invention)

Among the conventional hemostatic measures is hemostasis with a fibrin adhesive. Specifically, a fibrin adhesive is used for adhesion, sealing and hemostasis of tissues by overlaying fibrin and thrombin solutions on wounded regions or by applying a mixed solution of fibrin and thrombin with a spraying device. However, using a fibrin adhesive alone for hemostasis of arterial bleeding, which is vigorous and may lead to much loss of blood, efficacious hemostasis is scarcely possible since said mixed ingredients in liquid may tend to be flowed away by the blood flow.

There are attempts to fix the ingredients of a fibrin adhesive onto a variety of substrates to thereby produce a sheet-like preparation. For such substrates, bioabsorbable/biodegradable materials have been used including natural components such as gelatin or collagen, or synthetic high molecular weight materials such as polyethylene glycol or polyglycolic acid. As an exemplary, a sheet preparation has been put into practice wherein horse-derived collagen holds fibrin and thrombin (e.g. Japanese patent publication No. 34830/1986). However, the substrate collagen of this sheet preparation is rather thick and somewhat rigid to render the sheet preparation poorly stick to wounded regions where hemostasis is desired, thereby making efficacious hemostasis sometimes difficult. Besides, said sheet preparation is such that the substrate is made of equine collagen and thrombin is derived from bovine, i.e. material derived from non-human animal species is used, and hence there is a possibility of induction of an antibody against heterologous proteins or onset of zoonotic infections such as prion disease, being far from ideal one. On the other hand, the use of a fibrin adhesive for hemostasis will advantageously induce a fewer reaction to foreign substance but has the problems as previously described.

In order to solve these problems, one approach is to develop a fibrin adhesive that may permit hemostasis in a short time, suppress a loss of blood to a minimum extent and allow for thorough hemostasis. Such a fibrin adhesive will be required to consist of the same coagulation factor as in human free from infectious agents, to be in the form of a sheet so that hemostatic effect may fully be exerted, and to use a sheet made of a material strictly selected and devised to be safe to the living body.

### (Means for Solving the Problems)

In view of the above-mentioned various problems, the present inventors have carried out intensive investigation and as a consequence found that a hemostatic material comprising as an effective ingredient thrombin and fibrinogen characterized in that a bioabsorbable synthetic nonwoven fabric, which is a bioabsorbable synthetic material processed in the form of a nonwoven fabric, is used as a supporting material may exert quite excellent hemostatic effects, to thereby complete the present invention.

### (More Efficacious Effects than Prior Art)

The hemostatic material according to the present invention has excellent properties as listed below and hence is an ideal topical hemostatic material:
(1) It may be applied to bleeding under various conditions, including projectile bleeding and exudative bleeding, suppress a loss of blood to a minimum extent, and provide a thorough hemostasis;
(2) It is highly safe;
(3) It is absorbed with a lapse of time;
(4) It shows an excellent elasticity and flexibility;
(5) It allows for hemostasis at a broad area;
(6) It induces a slight or no inflammation reaction.

In accordance with the present invention, it is now possible to provide for a hemostatic material comprising a bioabsorbable synthetic nonwoven fabric which enables safe, prompt and thorough hemostasis in various clinical fields, typically in a surgical operation in various fields of the operation.

### BEST MODE FOR CARRYING OUT THE INVENTION

The bioabsorbable synthetic nonwoven fabric for use in the present invention may be any nonwoven fabric made of a bioabsorbable synthetic fiber. A bioabsorbable synthetic fiber as used herein refers to a synthetic fiber that is unlikely to induce inflammation in the living body as a foreign substance and may be absorbed and/or degraded within the living body with a lapse of time. The nonwoven fabric has preferably appropriate flexibility and elasticity to ensure that it may surely be stuck to any affected area. For example, a synthetic fiber that may form such a nonwoven fabric includes polyglycolic acid, polylactic acid, or a copolymer of glycolic acid with lactic acid, etc., which may be used after processing into a nonwoven fabric. Among these, a bioabsorbable synthetic nonwoven fabric which is prepared from polyglycolic acid by processing into a nonwoven fabric is the most preferable material for the purpose of the present invention.

The nonwoven fabric may be in any shape but preferably in the form of a sheet in view of versatility to various applications.

In addition to the effective ingredients, a pharmaceutically acceptable stabilizer and additive may also be added. Examples of such stabilizer and additive include, for instance, Factor XIII preferably derived from human blood or obtained by the genetic recombination technique, calcium chloride, a protease inhibitor (e.g. aprotinin), albumin, aminoacetic acid, polyethylene glycol, arginine, sodium hyaluronate, glycerol, mannitol, and the like.

Thrombin, fibrinogen and Factor XIII may preferably be derived from human blood or obtained by the genetic recombination technique.

The hemostatic material of the present invention may be in any dosage form so far as thrombin and fibrinogen as an effective ingredient are ultimately contained in a bioabsorbable synthetic nonwoven fabric.

In view of easy handling under operative settings, however, a bioabsorbable synthetic nonwoven fabric previously holding thrombin, which maintains flexibility, is one of preferable embodiments from the viewpoint of its easy handling as well as hemostatic efficacy.

In case that a bioabsorbable synthetic nonwoven fabric previously holds both thrombin and fibrinogen, the nonwoven fabric should hold each of thrombin and fibrinogen under such condition that the components are separated from each other or each of the components in the form of powder are suspended in an organic solvent and each suspension is sprayed to the nonwoven fabric, so that both thrombin and fibrinogen may not react to each other before use to generate stabilized fibrin.

The hemostatic material of the present invention may be formulated as a kit comprising either:
(i) a bioabsorbable synthetic nonwoven fabric holding thrombin plus fibrinogen; or
(ii) a bioabsorbable synthetic nonwoven fabric, thrombin, and fibrinogen;
in which a stabilizer and an additive as described above may optionally be added to both (i) and (ii).

For use in case of (i), a bioabsorbable synthetic nonwoven fabric holding thrombin is immersed into a solution containing fibrinogen, or said solution is sprayed to the bioabsorbable synthetic nonwoven fabric holding thrombin. Said bioabsorbable synthetic nonwoven fabric holding thrombin may be prepared by (1) dissolving thrombin in a saline or a buffer and optionally adding to the resulting thrombin solution calcium chloride as an additive, and (2) immersing a bioabsorbable synthetic nonwoven fabric into said thrombin solution, followed by freezing at -80°C for 2 hours and lyophilization.

For use in case of (ii), a solution containing thrombin and a solution containing fibrinogen are prepared as in the process for preparing a commercially available fibrin adhesive (e.g. Bolheal manufactured by Juridical Foundation The Chemo-Sero-Therapeutic Research Institute) and a bioabsorbable synthetic nonwoven fabric is then immersed into the solutions of thrombin and fibrinogen successively, or each of the solutions of thrombin and fibrinogen is applied simultaneously via spray.

In either case of (i) or (ii), Factor XIII or a protease inhibitor may be added to a solution containing fibrinogen.

The hemostatic material obtained in accordance with the present invention, due to its high adhesiveness, appropriate strength, flexibility and elasticity, may be stuck to bleeding regions in any shape, allowing for prompt hemostasis for various bleeding conditions such as projectile bleeding and exudative bleeding.

Polyglycolic acid bioabsorbable nonwoven fabric as used for the substrate in the hemostatic material of the present invention is highly safe since it is absorbed within the living body and degraded into water and carbon dioxide.

As such, the hemostatic material according to the present invention may easily and quickly be applied to topical bleeding and allow for efficient hemostasis through both pressure and a blood coagulation reaction. Besides, since every material used therein is safe to the living body, it may be used in clinical settings without care.

The present invention is explained in more detail by means of the following Examples but should not be construed to be limited thereto.

### Example 1: Preparation of sheet holding thrombin

A sheet holding thrombin in accordance with the present invention was prepared by the process as described below.

To a solution containing 5% human serum albumin are added 40 mM calcium chloride and subsequently thrombin derived from human blood at a final concentration of 500 U/mL. The solution is poured into a vessel at a depth of 1 mm where a bioabsorbable synthetic nonwoven fabric made of polyglycolic acid (Neoveil, Gunze Limited, thickness 0.15 mm) is laid on the bottom. The sheet, after being frozen at -80°C for 2 hours and lyophilized, is used as a sample of a sheet holding thrombin (thrombin held at 50 U/cm²).

### Example 2: Test for hemostasis in projectile bleeding

Hemostatic effect to projectile bleeding was investigated for a combination of the sheet holding thrombin as prepared in Example 1 and a fibrinogen solution.

Assessment used is indicated below.
(1) Test rabbit was subject to abdominal section under anesthesia with Nembutal (20 to 35 mg/kg).
(2) Heparin was intravenously administered at 300 U/kg.
(3) The abdominal aorta was stuck with 21G needle to generate projectile bleeding.
(4) Attempt was made to cease bleeding for each of Groups with various hemostatic means as described below. Hemostasis was conducted while blood spouting.

### Group 1: Sheet holding thrombin + fibrinogen solution

A fibrinogen solution (Bolheal manufactured by Juridical Foundation The Chemo-Sero-Therapeutic Research Institute, "A solution"; about 0.7 mL) was sprayed to the sheet holding thrombin (2×2cm) prepared in Example 1, which was immediately placed onto the bleeding region and lightly pressed for 1 minute.

### Group 2: Polyglycolic acid nonwoven fabric + fibrin adhesive

To the polyglycolic acid bioabsorbable synthetic nonwoven fabric (Neoveil, Gunze Limited, 2×2cm) as used in Example 1 as a substrate was sprayed a fibrin adhesive (Bolheal, Juridical Foundation The Chemo-Sero-Therapeutic Research Institute, a solution containing fibrinogen ("A solution") and a solution containing thrombin ("B solution"); each about 0.7 mL), which was immediately placed onto the bleeding region and lightly pressed for 1 minute.

### Group 3: Collagen sheet preparation

A collagen sheet preparation in which components of a fibrin adhesive are fixed (TachoComb, Torii Pharmaceutical Co., Ltd.; fibrinogen and thrombin components are fixed by lyophilization on one side of a sponge sheet made of equine collagen as a supporting material: 2×2cm) was placed onto the bleeding region and lightly pressed for 1 minute.
(5) After hemostatic treatment, whether hemostasis was attained or not was determined. When bleeding still occurred, the same hemostatic treatment was repeated for at most three times.
(6) After completion of hemostasis, if there is recurrence of bleeding was observed for five minutes.

As a result, as shown in Table 1, Group 1 of a combination of a sheet holding thrombin and a fibrinogen solution could thoroughly cease projectile bleeding with a single hemostatic treatment in this model. Group 2 of a polyglycolic acid nonwoven fabric to which a fibrin adhesive was sprayed was also proved to exhibit an excellent hemostatic effect though at a slightly lower level than that of Group 1 as having a case where bleeding could not be ceased with a single hemostatic treatment. On the contrary, with a collagen sheet of Group 3, bleeding could be ceased with a single hemostatic treatment in only few cases. Recurrence of bleeding after completion of hemostasis was observed in neither of Groups 1 to 3.

**Table 1**

| Group | Nos. of bleeding regions | Nos. of treatments | | |
|---|---|---|---|---|
| | | 1st | 2nd | 3rd |
| 1 | 7 | 7 | 0 | 0 |
| 2 | 7 | 6 | 1 | 0 |
| 3 | 7 | 2 | 3 | 2 |

### Example 3: Test for hemostasis in exudative bleeding

Hemostatic effect to exudative bleeding (oozing) was investigated for a combination of the sheet holding thrombin prepared in Example 1 and a fibrinogen solution.

Assessment used is indicated below.
(1) Test rabbit was subject to abdominal section under anesthesia with Nembutal (20 to 35 mg/kg).
(2) Heparin was intravenously administered at 300 U/kg.
(3) The right lobe, the inner left lobe or the outer left lobe of the liver was wounded in circle to thickness of 4 mm with a leather punch of 1.5 cm diameter and the wounded region was excised with a surgical knife.
(4) Bleeding from the wound was absorbed with gauze for 10 seconds and weighed (0.68 g). No difference in an amount of bleeding was observed among Groups.
(5) Hemostatic treatment was performed in the same Groups 1 to 3 as in Example 2.
(6) Bleeding for 5 minutes, including the time required for the hemostatic treatment, was absorbed with gauze and weighed. When bleeding from the wound surface was observed after 5 minutes, the hemostatic treatment and the weighing of bleeding were repeated.
(7) The hemostatic treatment was repeated for at most three times and assessment was made with a frequency of the hemostatic treatment needed for hemostasis and a total weight of bleeding from the initiation of the hemostatic treatment up till hemostasis.

As a result, as shown in Table 2, Group 1 of a combination of a sheet holding thrombin and a fibrinogen solution could completely cease exudative bleeding with a single hemostatic treatment in this model and bleeding after hemostatic treatment (an amount of bleeding up till hemostasis) could be suppressed to an extremely low level. With a polyglycolic acid nonwoven fabric to which a fibrin adhesive was sprayed of Group 2, exudative bleeding could be ceased with a single hemostatic treatment although a total bleeding after hemostatic treatment was observed at somewhat higher level than the combination of a sheet holding thrombin and a fibrinogen solution. On the contrary, with a collagen sheet of Group 3, bleeding could not be ceased with a single hemostatic treatment and there were even cases where bleeding could not be ceased with three hemostatic treatments. Moreover, a high level of a total bleeding after hemostatic treatment was observed.

**Table 2**

| Group | Nos. of bleeding regions | Nos. of hemostatic treatments | | | Total bleeding after hemostatic treatments (g/5 min. x Nos. of treatments) |
|---|---|---|---|---|---|
| | | 1st | 2nd | 3rd | |
| 1 | 7 | 7 | 0 | 0 | 0.05 ± 0.033 |
| 2 | 5 | 5 | 0 | 0 | 0.11 ± 0.055 |
| 3 | 5 | 0 | 3 | 2* | 6.41 ± 5.736 |

| | | | | | |
|---|---|---|---|---|---|
| *: Even after the third hemostatic treatment, hemostasis was not possible. | | | | | |

The bioabsorbable synthetic nonwoven fabric coated with fibrinogen and thrombin according to the present invention could provide thorough hemostasis with hemostatic treatment for as short as 1 minute as shown in Examples 2 and 3.

## Claims

1. A hemostatic material comprising as an effective ingredient thrombin and fibrinogen **characterized in that** a bioabsorbable synthetic nonwoven fabric is used as a supporting material.

2. The hemostatic material according to claim 1, wherein said bioabsorbable synthetic nonwoven fabric is made of a material selected from the group consisting of polyglycolic acid, polylactic acid and a copolymer of glycolic acid and lactic acid.

3. The hemostatic material according to claim 1 or 2, wherein said bioabsorbable synthetic nonwoven fabric is a nonwoven fabric made of a material of polyglycolic acid.

4. The hemostatic material according to any of claims 1 to 3, wherein the bioabsorbable synthetic nonwoven fabric previously holds at least thrombin among thrombin and fibrinogen.

5. The hemostatic material according to any of claims 1 to 4, wherein said hemostatic material comprises at least one additive selected from Factor XIII, a protease inhibitor, or calcium chloride.

6. The hemostatic material according to any of claims 1 to 5, wherein thrombin, fibrinogen and Factor XIII are either derived from human blood or produced by a genetic recombination technique.

7. Use of a combination of a bioabsorbable synthetic nonwoven fabric as a supporting material and thrombin and fibrinogen as an effective ingredient for a hemostatic material.

8. The use according to claim 7, wherein said bioabsorbable synthetic nonwoven fabric is made of a material selected from the group consisting of polyglycolic acid, polylactic acid and a copolymer of glycolic acid and lactic acid.

9. The use according to claim 7 or 8, wherein said bioabsorbable synthetic nonwoven fabric is a nonwoven fabric made of a material of polyglycolic acid.

10. The use according to any of claims 7 to 9, wherein said hemostatic material comprises at least one additive selected from Factor XIII, a protease inhibitor, or calcium chloride.

11. The use according to claim 10, wherein said Factor XIII is added to fibrinogen.

12. The use according to any of claims 7 to 11, wherein said thrombin, fibrinogen and Factor XIII are either derived from human blood or produced by a genetic recombination technique.

13. Use of a combination of a bioabsorbable synthetic nonwoven fabric holding thrombin as an effective ingredient, and fibrinogen as an effective ingredient for a hemostatic material.

14. The use according to claim 13, wherein said bioabsorbable synthetic nonwoven fabric holding thrombin as an effective ingredient is prepared by the steps of immersing a bioabsorbable synthetic nonwoven fabric into a solution containing thrombin and of lyophilizing the obtained nonwoven fabric.

15. The use according to claim 13 or 14, wherein said bioabsorbable synthetic nonwoven fabric is made of a material selected from the group consisting of polyglycolic acid, polylactic acid and a copolymer of glycolic acid and lactic acid.

16. The use according to any of claims 13 to 15, wherein said bioabsorbable synthetic nonwoven fabric is a nonwoven fabric made of a material of polyglycolic acid.

17. The use according to any of claims 13 to 16, wherein said hemostatic material comprises at least one additive selected from Factor XIII, a protease inhibitor, or calcium chloride.

18. The use according to claim 17, wherein said calcium chloride is fixed to the bioabsorbable synthetic nonwoven fabric together with thrombin.

19. The use according to claim 17 or 18, wherein said Factor XIII is added to fibrinogen.

20. The use according to any of claims 13 to 19, wherein said thrombin, fibrinogen and Factor XIII are either derived from human blood or produced by a genetic recombination technique.

21. A hemostatic kit comprising a bioabsorbable synthetic nonwoven fabric holding thrombin as an effective ingredient, and a container comprising fibrinogen as an effective ingredient.

22. The hemostatic kit according to claim 21, wherein said bioabsorbable synthetic nonwoven fabric is made of a material selected from the group consisting of polyglycolic acid, polylactic acid and a copolymer of glycolic acid and lactic acid.

23. The hemostatic kit according to claim 21 or 22, wherein said bioabsorbable synthetic nonwoven fabric is a nonwoven fabric made of a material of polyglycolic acid.

24. The hemostatic kit according to any of claims 21 to 23, wherein said hemostatic kit comprises at least one additive selected from Factor XIII, a protease inhibitor, or calcium chloride.

25. The hemostatic kit according to claim 24, wherein said calcium chloride is added to the bioabsorbable synthetic nonwoven fabric as an additive for thrombin.

26. The hemostatic kit according to claim 24 or 25, wherein said Factor XIII is included in a container comprising fibrinogen.

27. The hemostatic kit according to any of claims 21 to 26, wherein said thrombin, fibrinogen and Factor XIII are either derived from human blood or produced by a genetic recombination technique.

28. The hemostatic kit according to any of claims 21 to 27, wherein said bioabsorbable synthetic nonwoven fabric holding thrombin is prepared by the steps of immersing a bioabsorbable synthetic nonwoven fabric into a solution containing thrombin and of lyophilizing the obtained nonwoven fabric.

29. A hemostatic kit comprising a bioabsorbable synthetic nonwoven fabric as a substrate, a container comprising thrombin as an effective ingredient and a container comprising fibrinogen as an effective ingredient.

30. The hemostatic kit according to claim 29, wherein said bioabsorbable synthetic nonwoven fabric is made of a material selected from the group consisting of polyglycolic acid, polylactic acid and a copolymer of glycolic acid and lactic acid.

31. The hemostatic kit according to claim 29 or 30, wherein said bioabsorbable synthetic nonwoven fabric is a nonwoven fabric made of a material of polyglycolic acid.

32. The hemostatic kit according to any of claims 29 to 31, wherein said hemostatic kit comprises at least one additive selected from Factor XIII, a protease inhibitor, or calcium chloride.

33. The hemostatic kit according to claim 32, wherein said Factor XIII is included in a container comprising fibrinogen.

34. The hemostatic kit according to any of claims 29 to 33, wherein said thrombin, fibrinogen and Factor XIII are either derived from human blood or produced by a genetic recombination technique.
